# EUROPEAN PATENT APPLICATION

(11) **EP 4 321 122 A1**
(43) Date of publication of application: **14.02.2024**
(21) Application number: 23190639.7
(22) Date of filing: 09.08.2023
(51) Int. Cl.: A61B 90/00

(54) **EXTERNAL FIXATION FRAME REFERENCE BODIES**

(30) Priority: 12.08.2022 US 202263397510 P
(71) Applicant: Stryker European Operations Limited, Carrigtwohill, Co. Cork T45 HX08 (IE)
(72) Inventor: SHAH, Gretchen, Wayne, 07470 (US); MANNANAL, Subash K., Boonton, 07005 (US); ALANIS, Melisa, Hackensack, 07601 (US); BRAGG, William, Eads, 38028 (US); MOORE, Jesse, Germantown, 38138 (US); ROBINSON, Christopher, Hernando, 38632 (US)
(74) Representative: Regimbeau

(57) **Abstract**

Disclosed herein are reference bodies for an external fixation frame and methods for using reference bodies for external fixation frame placement and arrangement. An external fixation frame according to the present disclosure may include first and second support rings, one or more wires coupled to any of the first and second support rings, one or more struts extending between the first and second support rings, and a reference body including a plurality of markers. The reference body may contact the one or more wires or the one or more struts. The plurality of markers may be arranged in a pattern to identify an orientation of the external fixation frame.

## Description

### CROSS-REFERENCE TO RELATED APPLICATION

This application claims the benefit of the filing date of United States Provisional Application No. 63/397,510 filed on August 12, 2022, the disclosure of which is hereby incorporated by reference in its entirety.

### FIELD OF INVENTION

The present disclosure relates to reference bodies for surgical devices and methods for using reference bodies for surgical device placement, and particularly to reference bodies for external fixation frames and methods for using reference bodies for external fixation frame placement and arrangement.

### BACKGROUND OF THE INVENTION

Various orthopedic external fixation procedures such as a bone deformity correction, fracture reduction, limb lengthening, etc., require an external fixation frame to be properly positioned and coupled to a patient's bone. External fixation frames are not only required to remain stably fixed to the target surgical site, but are also required to be adjusted multiple times during the procedure to vary orientation and positions of the various components of the external fixation frames with reference to the target surgical site. For example, struts extending between support rings of an external fixation frame may need to be adjusted daily by precise length increments or decrements during patient recovery.

Proper placement and orientation of the external fixation frame is generally determined from radiographic images. For example, the support rings of the external fixation frame may need to be perpendicular to the patient's bone while coupled to the patient. Generally, two perpendicular 2D radiographic images, an anterior-posterior image and a medial lateral image, of the external fixation frame at the surgical site are used in surgical planning. Multiple radiographic reference bodies can be placed on the external fixation frames to aid in verifying image orientation. However, positioning a patient and the external fixation frame with multiple reference bodies adjacent a single X-ray source to obtain two precisely perpendicular images can be challenging and often result in alignment inaccuracies. These alignment inaccuracies may impact surgical planning and consequently lead to improper positioning of the external fixation frame. Further, ensuring all of the various components of the external fixation frame are properly positioned with respect to the bone and each other after each daily adjustment can be challenging.

Therefore, there exists a need for improved external fixation frames and methods for using reference bodies for external fixation frame placement and arrangement.

### BRIEF SUMMARY OF THE INVENTION

Disclosed herein are reference bodies for surgical devices and methods for using reference bodies for surgical device placement and adjustment.

In accordance with an aspect of the present disclosure, an external fixation frame is provided. An external fixation frame according to this aspect, may include first and second support rings, one or more wires and/or bone pins coupled to any of the first and second support rings, one or more struts extending between the first and second support rings, and a reference body including a plurality of markers. The reference body may contact any of the one or more wires and/or bone pins or the one or more struts. The plurality of markers may be arranged in a pattern to identify an orientation of the external fixation frame.

Continuing in accordance with this aspect, the orientation of the external fixation frame may be with respect to an adjacent bone. The external fixation frame may be coupled to the bone.

Continuing in accordance with this aspect, the plurality of markers may be radiopaque. An orientation of the external fixation frame with reference to the bone in an image may be determined from the pattern.

Continuing in accordance with this aspect, the plurality of markers may define a data code. The data code may be any of a linear barcode, a matrix code, and a Quick Response (QR) code.

Continuing in accordance with this aspect, the plurality of markers may be tracking markers configured to be tracked by a camera.

Continuing in accordance with this aspect, a scale of the external fixation frame with reference to the bone in an image is determined from the pattern.

Continuing in accordance with this aspect, the reference body may define a cuboid. The plurality of markers may be spherical bodies disposed at least partially within the reference body. The plurality of markers may be cylindrical bodies. The cylindrical bodies may include threads. A first set of cylindrical bodies may extend from a first face of the cuboid and a second set of cylindrical bodies may extend from a second face of the cuboid.

Continuing in accordance with this aspect, the reference body may include an opening to receive the one or more wires. The opening may define a longitudinal axis extending parallel to the one or more wires. The opening may define a first dimension smaller than a second dimension. A diameter of the one or more wires may be less than the first dimension. The reference body may include an alignment opening configure to align the external fixation frame with reference to an imaging device.

In accordance with another aspect of the present disclosure, an external fixation frame is provided. An external fixation frame according to this aspect, may include first and second support rings, one or more wires and/or bone pins extending from a bone to the first or second support ring, one or more struts extending between the first and second support rings, and a reference body including a plurality of markers and a snap-fit connector. The reference body may be attachable to any of the first and second support rings and/or the one or more struts via the snap-fit connector. The plurality of markers may be arranged in a pattern to identify an orientation of the external fixation frame with reference to the bone.

Continuing in accordance with this aspect, the snap-fit connector may include first and second spaced apart legs configure to be received in a corresponding opening of the first and second support rings or the one or more struts. The snap-fit connector may define an arcuate sleeve having an opening. The opening may define a first dimension less than a diameter of the one or more struts.

Continuing in accordance with this aspect, the plurality of markers are radiopaque. An orientation of the external fixation frame with reference to the bone in an image may be determined from the pattern.

Continuing in accordance with this aspect, the plurality of markers may define a data code. The data code may be any of a linear barcode, a matrix code, and a Quick Response (QR) code.

Continuing in accordance with this aspect, the plurality of markers may be tracking markers configured to be tracked by a camera.

Continuing in accordance with this aspect, a scale of the external fixation frame with reference to the bone in an image may be determined from the pattern.

Continuing in accordance with this aspect, the reference body may include a cuboid body. The plurality of markers may be spherical bodies disposed at least partially within the cuboid body. The cuboid body may be radiolucent. The plurality of markers may be cylindrical bodies. The cylindrical bodies may include threads. A first set of cylindrical bodies may extend from a first face of the cuboid body and a second set of cylindrical bodies may extend from a second face of the cuboid body.

In accordance with another aspect of the present disclosure, an external fixation frame is provided. An external fixation frame according to this aspect, may include first and second support rings, one or more wires and/or bone pins extending from a bone to the first or second support ring, one or more struts extending between the first and second support rings, and a marker defined by a framework of radiopaque elements. The marker may be attachable to the external fixation frame. The framework may be arranged in a pattern to identify an orientation of the external fixation frame with reference to the bone.

Continuing in accordance with this aspect, the framework may define a lattice.

Continuing in accordance with this aspect, an orientation of the external fixation frame with reference to the bone in an image may be determined from the pattern.

Continuing in accordance with this aspect, a scale of the external fixation frame with reference to the bone in an image may be determined from the pattern.

Continuing in accordance with this aspect, the framework may define a cuboid.

Continuing in accordance with this aspect, an additive manufacturing process may be used to create the framework.

In accordance with another aspect of present disclosure a method for orienting an external fixation frame with reference to a bone is provided. A method according to this aspect, may include the steps of positioning an external fixation frame about a bone, coupling a reference body to the external fixation frame, obtaining an image of the external fixation frame and the bone, and determining an orientation of the external fixation frame with reference to the bone from a pattern in the image. The reference body may include plurality of markers arranged in the pattern to identify an orientation of the external fixation frame with reference to the bone.

In accordance with another aspect of the present disclosure, a method for determining an orientation of an external fixation frame with reference to an imager is provided. A method according to this embodiment, may include the steps of positioning an external fixation frame adjacent an imager, coupling a reference body to the external fixation frame, the reference body may include a framework defining at least one shape, obtaining a first image of the external fixation frame and the at least one shape, determining a first value of a geometric property of the at least one shape from the image, and comparing the first value with a second value of the geometric property of the at least one shape to determine an orientation of the external fixation frame with reference to the imager.

Continuing in accordance with this aspect, the at least one shape may be a 2D shape. The 2D shape may be any of a triangle, rectangle, pentagon, trapezoid, hexagon, circle, sphere and rhombus. The first and second values of the geometric property of the at least one shape may be any of a length, angle and perimeter.

### BRIEF DESCRIPTION OF THE DRAWINGS

A more complete appreciation of the subject matter of the present disclosure and the various advantages thereof can be realized by reference to the following detailed description, in which reference is made to the following accompanying drawings:
FIG. 1 is a front view of an external fixation frame with a reference body according to an embodiment of the present disclosure;
FIG. 2 is an isometric view of a reference body according to another embodiment of the present disclosure;
FIG. 3 is a front view of a reference body according to another embodiment of the present disclosure;
FIG. 4A is an isometric view of a reference body according to another embodiment of the present disclosure;
FIG. 4B is a front view of markers of the reference body of FIG. 4A;
FIG. 5 is a partial transparency view of a reference body according to another embodiment of the present disclosure;
FIG. 6 is an isometric view of a reference body according to another embodiment of the present disclosure;
FIG. 7 is a partial transparency view of a reference body according to another embodiment of the present disclosure;
FIG. 8A is top view of a reference body according to another embodiment of the present disclosure;
FIG. 8B is a side view of the reference body of FIG. 8A;
FIG. 9A is front view of a reference body according to another embodiment of the present disclosure;
FIG. 9B is a front view of the reference body of FIG. 9A with a fastener;
FIG. 10 is a front view of a reference body according to another embodiment of the present disclosure;
FIG. 11 is a front view of a reference body according to another embodiment of the present disclosure;
FIG. 12 is an isometric view of a reference body according to another embodiment of the present disclosure;
FIG. 13A is front view of a reference body according to another embodiment of the present disclosure;
FIG. 13B is bottom view of the reference body of FIG. 13A;
FIG. 14 is a front view of a reference body according to another embodiment of the present disclosure;
FIG. 15 is a front view of a reference body according to another embodiment of the present disclosure;
FIG. 16 is a front view of a reference body according to another embodiment of the present disclosure;
FIG. 17 is a side view of the reference body of FIG. 16;
FIG. 18A is a top view of the reference body of FIG. 16 and the reference body of FIG. 8A;
FIG. 18B is a bottom view of the reference body of FIG. 16 and the reference body of FIG. 8A;
FIG. 19 is a side view of a reference body according to another embodiment of the present disclosure;
FIG. 20 is a top view of the reference body of FIG. 19;
FIG. 21 is a bottom view of the reference body of FIG. 19;
FIG. 22 is a front view of a reference body according to another embodiment of the present disclosure;
FIG. 23 is a front view of a reference body according to another embodiment of the present disclosure;
FIG. 24 is top view of an implant with a reference body according to an embodiment of the present disclosure;
FIG. 25 is a top view of an implant with a reference body according to another embodiment of the present disclosure;
FIG. 26 is an isometric view of a reference body in a first position according to an embodiment of the present disclosure;
FIG. 27 is an isometric view of the reference body of FIG. 26 in a second position;
FIG. 28 is an isometric view of a reference body according to an embodiment of the present disclosure;
FIG. 29 is a first side view of the reference body of FIG. 28, and
FIG. 30 is a second side view of the reference body of FIG. 28.

### DETAILED DESCRIPTION

Reference will now be made in detail to the various embodiments of the present disclosure illustrated in the accompanying drawings. Wherever possible, the same or like reference numbers will be used throughout the drawings to refer to the same or like features within a different series of numbers (*e.g.*, 100-series, 200-series, etc.). It should be noted that the drawings are in simplified form and are not drawn to precise scale. Additionally, the term "a," as used in the specification, means "at least one." The terminology includes the words above specifically mentioned, derivatives thereof, and words of similar import. Although at least two variations are described herein, other variations may include aspects described herein combined in any suitable manner having combinations of all or some of the aspects described.

As used herein, the terms "orientation," "alignment" and "placement" will be used interchangeably and as such, unless otherwise stated, the explicit use of either term is inclusive of the other term.

In describing preferred embodiments of the disclosure, reference will be made to directional nomenclature used in describing the human body. It is noted that this nomenclature is used only for convenience and that it is not intended to be limiting with respect to the scope of the present disclosure. As used herein, when referring to bones or other parts of the body, the term "anterior" means toward the front part of the body or the face, and the term "posterior" means toward the back of the body. The term "medial" means toward the midline of the body, and the term "lateral" means away from the midline of the body. The term "superior" means closer to the head, and the term "inferior" means more distant from the head.

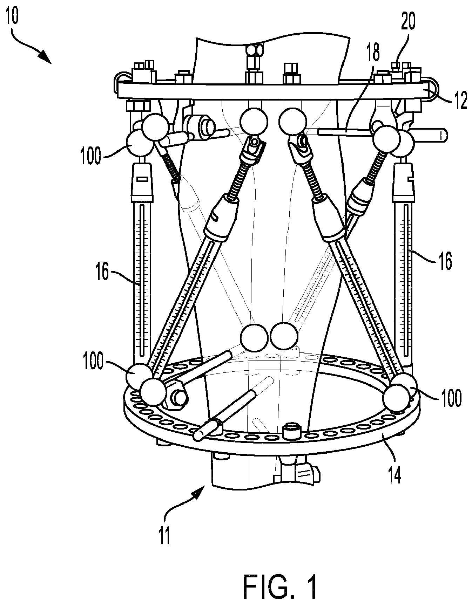

FIG. 1 is a front view of an exemplary external fixation frame 10 with multiple reference bodies 100 according to an embodiment of the present disclosure. External fixation frame 10 shown here is coupled to a patient's tibia 11 and can include multiple telescopic struts 16 extending between an upper support ring 12 and a lower support ring 14. Upper support ring 12 and lower support ring 14 are substantially flat such that each ring has a vertical cross section that is substantially rectangular. In other embodiments, support ring need not be flat, but rather can take on various shapes to accommodate other devices such as clamps, for example. A series of bone wires and/or bone pins 18 facilitate interaction between tibia 11 and external fixation frame 10.

As shown in FIG. 1, a plurality of reference bodies 100 are integrated to the U-joints of external fixation frame 10. The reference bodies can define any shape or size depending on the external fixation frame component they are integrated to form a monolithic component. In this embodiment, reference bodies 100 are spherical shaped bodies formed integrally with U-joints of the external fixation frame. Thus, external fixation frame 10 does not require distinct and separate reference bodies that need to be coupled and uncoupled with the external fixation frame to determine alignment and verify arrangement of the external fixation frame. Reference bodies 100 may include a radiolucent material such as a material that is entirely or partially transparent to radiation and/or almost entirely or partially transparent in X-ray, fluoroscopy, and other imaging modalities. Examples of radiolucent materials can include a polymers, plastic, para-aramid synthetic fiber, resins (e.g., polyether imide), carbon fiber or carbon composite, wood, cellulose, etc. Polymers can include polypropylene, polyethylene, polyether ether ketone, polyaryletherketone, acrylonitrile butadiene styrene, nylon, etc. Radiolucency of reference bodies 100 can be adjusted by combining material having varying densities within the reference bodies 100. For example, a reference body 100 can include a first material with a higher density than a second material of the reference body. This first material will absorb more radiation and be less radiolucent than the second material, which has a lower density and will absorb less radiation and be more radiolucent.

Reference bodies 100 can include a radiopaque material (not shown) arranged in a pattern or specific shape to determine orientation of the reference body and consequently orientation of external fixation frame 10. Radiopaque material pattern can be determined by the X-ray or other imaging modality, recognized by a camera or simply by a user's naked eye. The size of the reference bodies on the radiographic images relative to the other components of external fixation frame can be used to properly scale the obtained image. In addition to identifying the orientation and image scaling of the external fixation frame with the reference bodies, the location of reference bodies 100 - *i.e.,* on the ends of struts 16, allow a surgeon, patient, operator, or an imaging system to readily determine the length of each struts based via the associated reference body location. For example, an imaging camera can readily determine the position of reference bodies and transmit this information to an automated surgical planning software. The orientation and relative position of components can then be uploaded into a computational system for planning and optimizing bone correction as disclosed in U.S. Patent No. 8,654,150, the disclosure of which is hereby incorporated by reference herein.

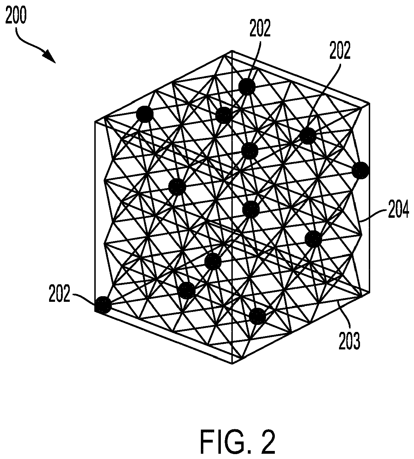

Referring now to FIG. 2, there is shown a reference body 200 according to another embodiment of the present disclosure. Reference body 200 includes an outer structure 204 made of partially or fully radiolucent material. Outer structure 204 can be formed by an additive manufacturing process to include a lattice frame 203 as shown in FIG. 2. Lattice frame 203 can be composed of material having a radiolucency different from the remainder of outer structure 204 to allow for orientation and image scaling. Angles defined between lattice frame edges can be used to identify reference body position. Markers 202 are positioned across and within outer structure 204 in specific patterns to allow for orientation and image scaling. Thus, a surgeon, patient, operator, or surgical planning software can utilize either lattice frame 203, markers 202 or both of a single reference body 200 to orient the external fixation frame. The lattice frame 203 and/or markers 202 of a single reference body provide sufficient reference points for proper alignment of the external fixation frame. Reference body 200 can be conveniently attached to any component of the external fixation frame such as the support rings, struts, bone wires, U-joints, etc., via an attachment (not shown) such as a clip, fastener, adhesive, etc.

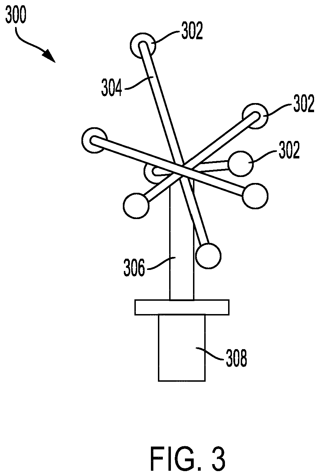

FIG. 3 shows a reference body 300 according to another embodiment of the present disclosure. Reference body 300 includes a plurality of extensions such as rods 304 with reference bodies 302 attached thereto. Varying rod lengths and angles between the rods allow for distinct reference body patterns that can be used for proper orientation of the external fixation frame components and positioning of the external fixation frame with respect to the target surgical site. A rod 306 connects rods 304 to an attachment 308 which can be readily coupled to any component of the external fixation frame. Rods 304 and/or markers 302 of a single reference body 300 provide sufficient reference points for proper alignment of the external fixation frame with reference to the target surgical site.

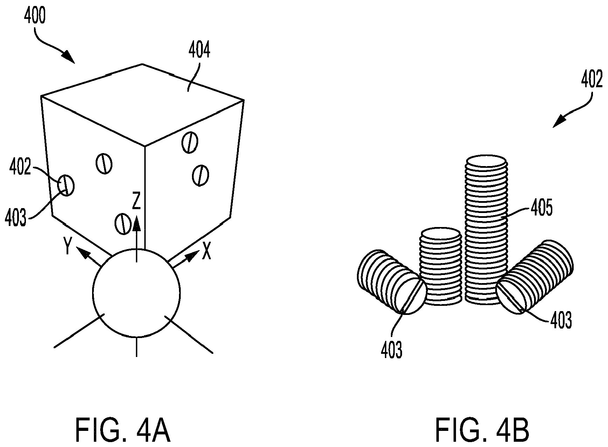

FIGS. 4A and 4B show a reference body 400 according to another embodiment of the present disclosure. Reference body 400 is similar to reference body 200, and therefore like elements are referred to with similar numerals within the 400-series of numbers. For example, reference body 400 includes an outer structure 404 with plurality of markers 402. However, outer structure 404 forms a solid structure with internally threaded openings to receive markers 402. Markers 402 are elongate bodies with external threads 405 configured to be threadingly engaged with openings in outer structure 404. A slot 403 on each marker forming a distinct pattern such as rectangle shown in FIG. 4B can be used to orient the external fixation frame. Outer structure 404 and markers 402 can be made of partially radiolucent material, radiopaque material or any combinations thereof. For example, a cuboidal metallic outer structure 404 can be drilled with multiple holes on various different sides to receive high-density plastic markers 402 which can be detected on X-ray or other imaging modules. Material properties of the outer structure and the markers can be varied to enhance radiographic visibility and visibility to the naked eye to facilitate external fixation frame alignment and orientation.

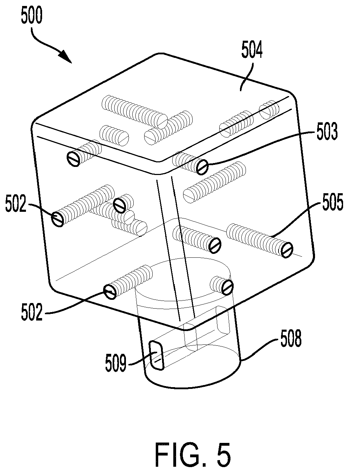

Referring now to FIG. 5, there is shown a reference body 500 according to another embodiment of the present disclosure. Reference body 500 is similar to reference body 400, and therefore like elements are referred to with similar numerals within the 500-series of numbers. For example, reference body 500 includes an outer structure 504 with plurality of markers 502. Reference body 500 includes an attachment extension 508 that can be readily coupled to any component of the external fixation frame. A slot 509 extending through the attachment extension provides additional imaging or visual cues for reference body 500 alignment and external fixation frame alignment. Markers 502 can be provided with varying colors to further facilitate reference body and external frame alignment.

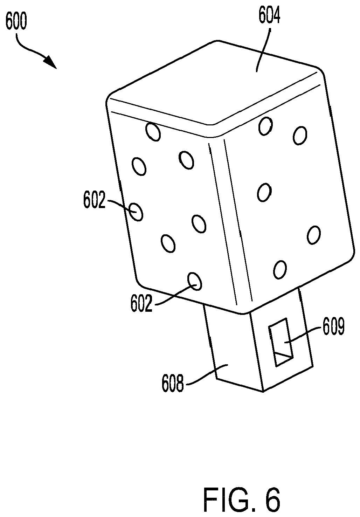

FIG. 6 shows a reference body 600 according to another embodiment of the present disclosure. Reference body 600 is similar to reference body 500, and therefore like elements are referred to with similar numerals within the 600-series of numbers. For example, reference body 600 includes an outer structure 604 with plurality of markers 602. However, markers 602 of reference body 600 are formed by indenting or gouging external surfaces of outer surface 604 to form hemispherical indentations as shown in FIG. 6. While markers consisting of hemispherical indentations are shown in this embodiment, other embodiments may include indentations of other shapes.

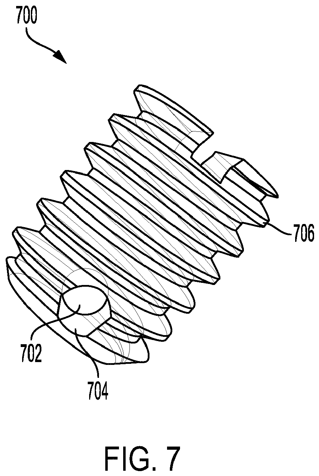

A reference body 700 according to an embodiment of the present disclosure is shown in FIG. 7. Reference body 700 includes a body comprising a threaded screw 706 with an opening 704 configured to receive and secure a marker 702. The threaded screw can be readily attached to various components of the external fixation frame such as the slots in the support rings, strut end caps, etc., to facilitate alignment and orientation.

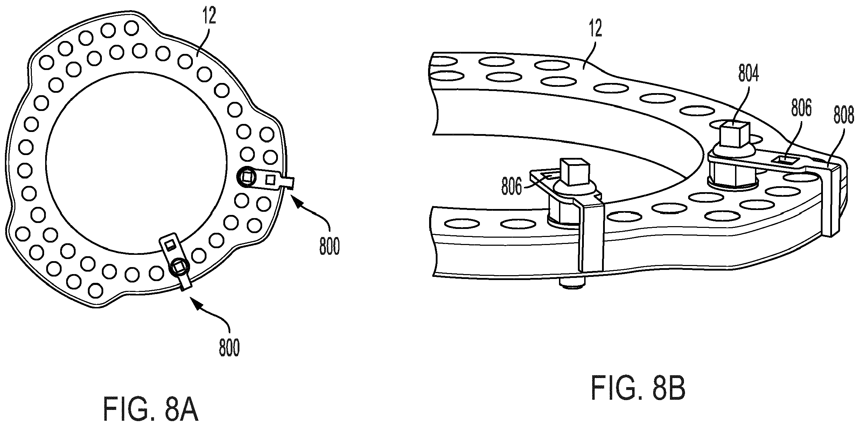

Referring now to FIGS. 8A and 8B, there is shown a reference body 800 coupled to upper support ring 12 according to another embodiment of the present disclosure. Reference body 800 is similar to reference body 500, and therefore like elements are referred to with similar numerals within the 800-series of numbers. For example, reference body 800 includes an outer structure 804 with plurality of markers 802 (not shown). Reference body 800 includes an L-shaped ledge 808 overhanging upper support ring 12 as best shown in FIG. 8B. A slot 806 extends through ledge 808. In addition to marker 802, ledge 808 and slot 806 facilitate reference body and external fixation frame alignment and orientation.

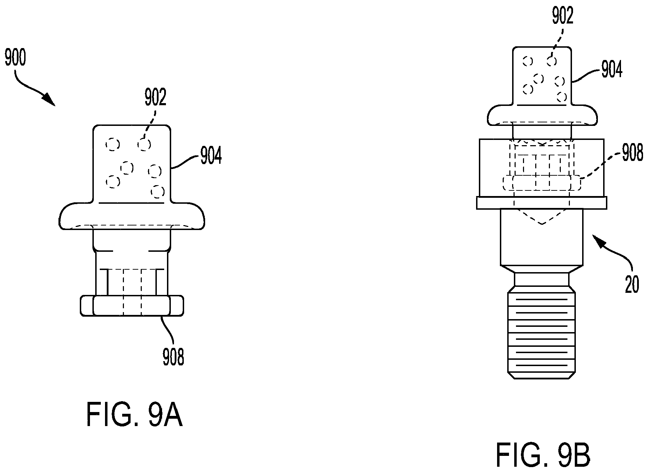

FIGS. 9A and 9B show a reference body 900 according to another embodiment of the present disclosure. Reference body 900 includes an outer structure 904 with marker 902. An attachment feature 908 provided at a distal end allows the reference body to be conveniently attached to or detached from a fastener 20 of the external fixation frame as best shown in FIG. 9B. In other embodiments, attachment feature 908 can be configured to be coupled with other components of the external fixation frame.

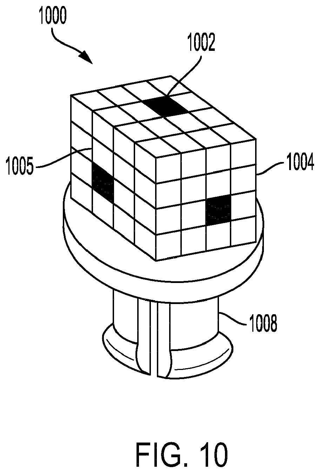

A reference body 1000 according to another embodiment of the present disclosure is shown in FIG. 10. Reference body 1000 is similar to reference body 900, and therefore like elements are referred to with similar numerals within the 1000-series of numbers. For example, reference body 1000 includes an outer structure 1004 with plurality of markers 1002. Markers 1002 of reference body 1000 are located within a grid pattern 1005 as shown in FIG. 10. In another embodiment, the reference body can include a QR code, bar code, data code, etc., that can be read by a scanner or camera to determine reference body and external fixation frame orientation. Attachment feature 1008 includes flexible legs which can be inserted into any component of the external fixation frame and secured via a snap fit.

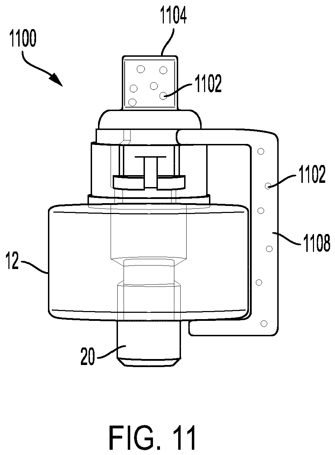

Referring now to FIG. 11, there is shown a reference body 1100 coupled to upper support ring 12 according to another embodiment of the present disclosure. Reference body 1100 is similar to reference body 900, and therefore like elements are referred to with similar numerals within the 1100-series of numbers. For example, reference body 1100 includes an outer structure 1104 with plurality of markers 1102. However, reference body 1100 includes a U-shaped ledge 1108 with additional markers 1102 located therein. This provides additional reference points for orienting and aligning reference body 1100 and external fixation frame 10. In another embodiment, outer structure 1104 can be attached to fastener 20. In another embodiment, ledge 1108 can be attached to both fastener 20 and upper support ring 12. In another embodiment, outer structure 1104 may retain ledge 1108 in a fixed position relative to fixation frame 10 and fastener 20.

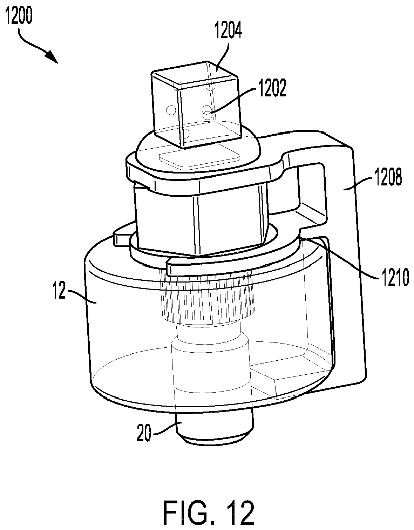

FIG. 12 shows a reference body 1200 coupled to upper support ring 12 according to another embodiment of the present disclosure. Reference body 1200 is similar to reference body 1100, and therefore like elements are referred to with similar numerals within the 1200-series of numbers. For example, reference body 1200 includes an outer structure 1204 with plurality of markers 1202. U-shaped ledge 1208 includes a ledge 1210 extending over upper support ring 12 as shown in FIG. 12.

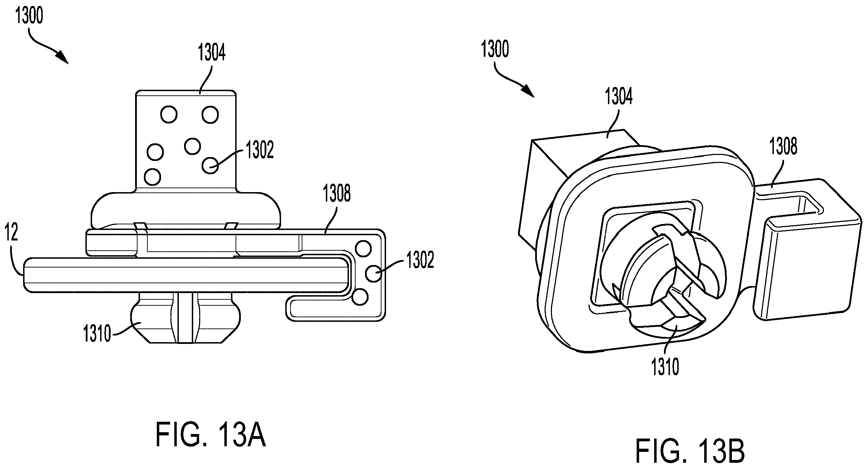

Referring now to FIGS. 13A and 13B, there is shown a reference body 1300 coupled to upper support ring 12 according to another embodiment of the present disclosure. Reference body 1300 is similar to reference body 1100, and therefore like elements are referred to with similar numerals within the 1300-series of numbers. For example, reference body 1300 includes an outer structure 1304 with plurality of markers 1302. However, reference body 1300 is configured to be coupled directly to upper support ring 12 via snap-fit connector 1310 as best shown in FIG. 13B.

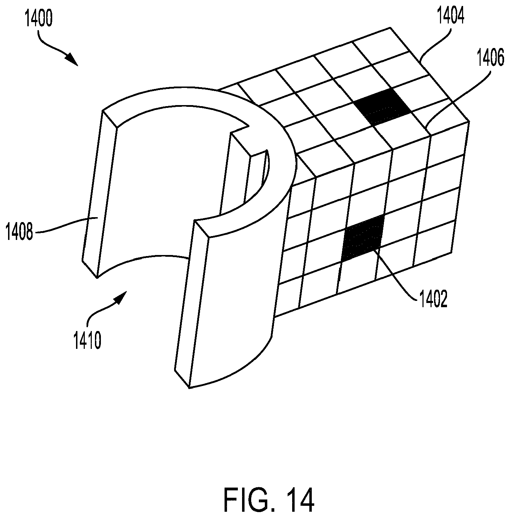

FIG. 14 shows a reference body 1400 according to another embodiment of the present disclosure. Reference body 1400 includes an outer structure 1404 with a plurality of markers 1402 located within a grid pattern 1406. A pair of flexible arms 1408 defining an opening 1410 are configured to readily couple reference body 1400 to various components of the external fixation frame such as struts 16.

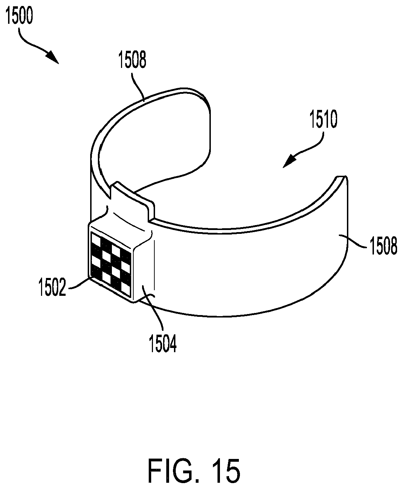

A reference body 1500 according to another embodiment of the present disclosure is shown in FIG. 15. Reference body 1500 is similar to reference body 1400, and therefore like elements are referred to with similar numerals within the 1500-series of numbers. For example, reference body 1500 includes an outer structure 1504 and a pair of flexible arms 1508 defining an opening 1510 configured to readily couple reference body 1500 to various components of the external fixation frame such as struts 16. However, reference body includes a QR code, bar code, data code, etc., that can be read by a scanner or camera to determine reference body and external fixation frame orientation as shown in FIG. 15.

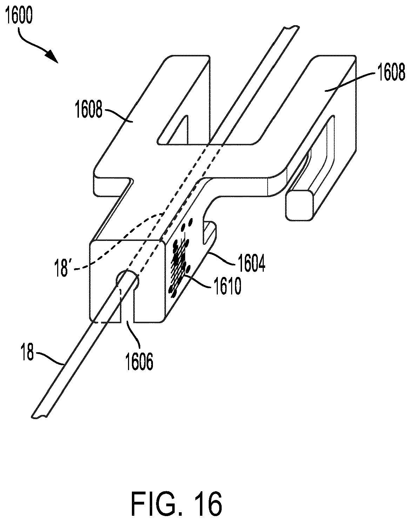

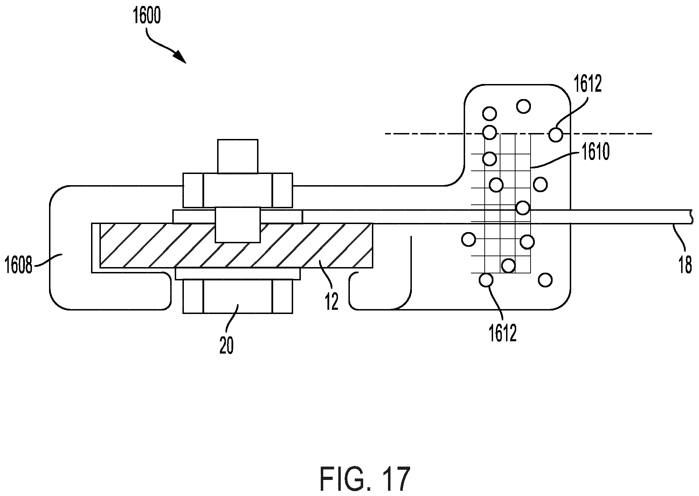

Referring now to FIGS. 16 and 17, there is shown a reference body 1600 coupled to a bone wire 18 and upper support ring 12 according to another embodiment of the present disclosure. Reference body 1600 includes an outer structure 1604 and a pair of arms 1608 configured to be coupled to upper support ring 12 as best shown in FIG. 17. A slot 1606 extending through the reference body is provided to receive and secure bone wire 18. Slot 1606 is shaped as keyhole slot to allow the bone wire to be inserted through the elongated section of slot 1606 and secured in the circular section of the slot as best shown in FIG. 16. The keyhole slot provides a reference pattern to allow for proper alignment and orientation of reference body 1600 and external fixation frame 10. A plurality of markers 1612 are disposed within a grid pattern 1610 on outer structure 1604. As shown in FIG. 17, reference body can be conveniently coupled to bone wire 18 and upper support ring 12 of external fixation frame. In addition to markers 1612, grid pattern 1610 and keyhole slot 1606, the position of the bone wire and/or upper support ring with reference to reference body 1600 can be used to orient and align external fixation frame 10.

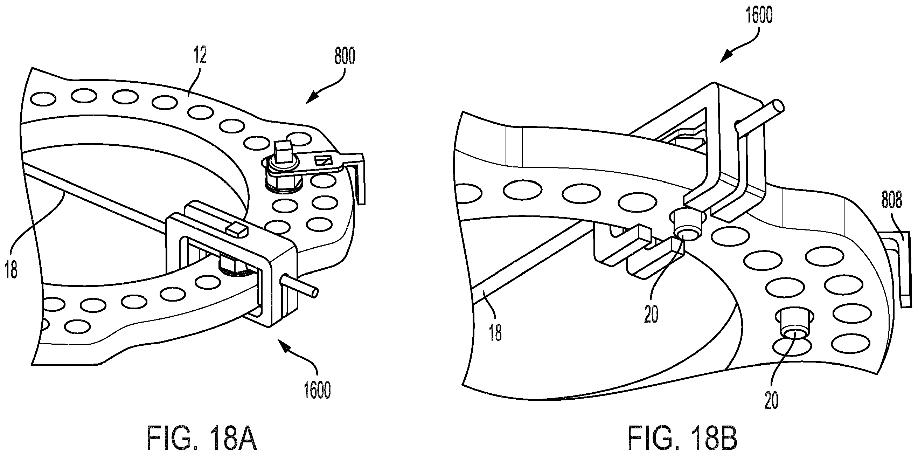

Any of the reference bodies disclosed herein can be used in combination with each other in external fixation frame 10. For example, FIG. 18A and 18B show support ring 12 coupled to reference body 800 and reference body 1600. Reference body 1600 is also coupled to bone wire 18. Depending on the type of external fixation frame or surgical device, multiple reference bodies disclosed herein can be combined to improve alignment, orientation, and arrangement of the surgical device.

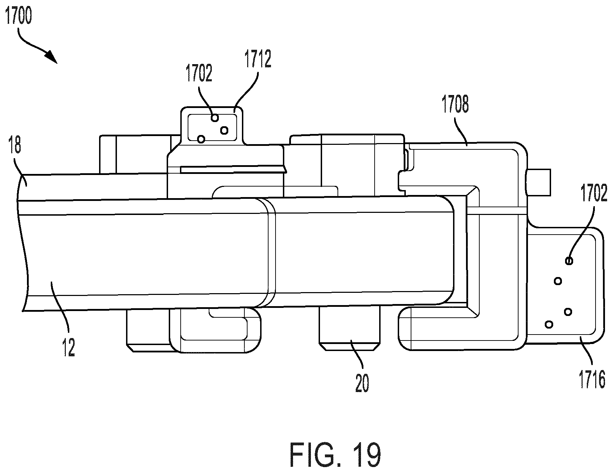

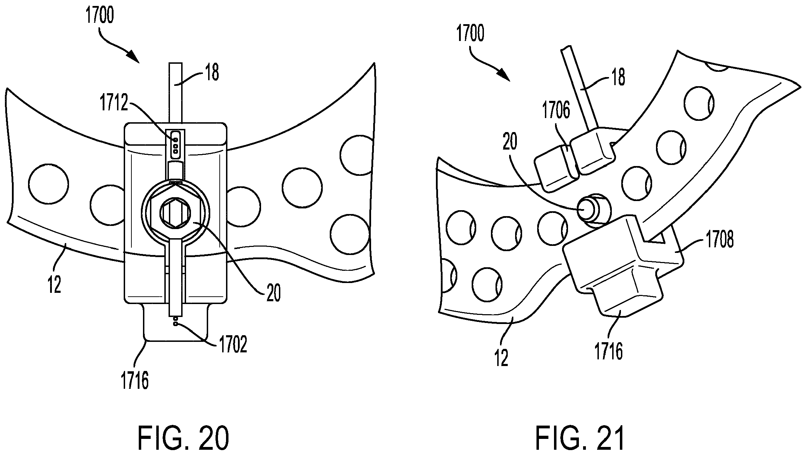

FIGS. 19-21 show a reference body 1700 according to another embodiment of the present disclosure. Reference body 1700 is similar to reference body 1600, and therefore like elements are referred to with similar numerals within the 1700-series of numbers. For example, reference body 1700 includes arms 1708 configured to be attached to upper support ring 12 and slot 1706 to receive and secure bone wire 18. However, reference body includes an upper structure 1712 and a side structure 1716 with markers 1712 as best shown in FIG. 19.

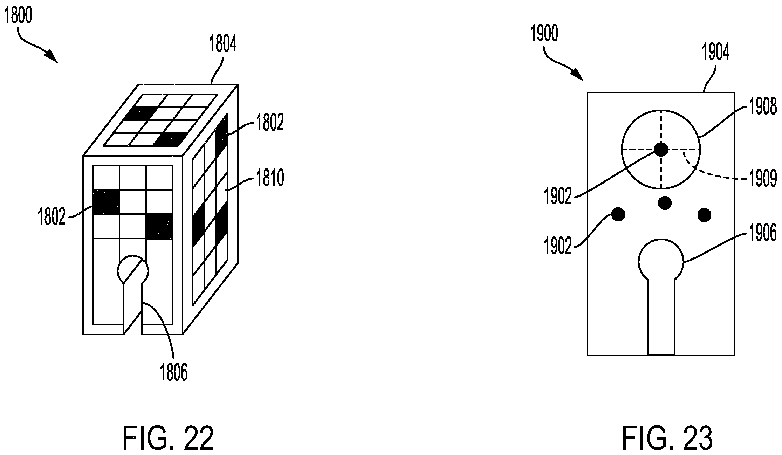

A reference body 1800 according to another embodiment of the present disclosure is shown in FIG. 22. Reference body 1800 is similar to reference body 1600, and therefore like elements are referred to with similar numerals within the 1700-series of numbers. For example, reference body 1800 includes a slot 1806 to receive and secure bone wire 18. However, reference body 1800 is only configured to be secured to bone wire 18 and does not include other attachment features and is therefore smaller in size than reference body 1600. Reference body 1800 includes a plurality of markers 1802 disposed within grid pattern 1810.

FIG. 23 shows a reference body 1900 according to another embodiment of the present disclosure. Reference body 1900 is similar to reference body 1800, and therefore like elements are referred to with similar numerals within the 1900-series of numbers. For example, reference body 1900 includes a slot 1906 to receive and secure bone wire 18. Reference body 1900 includes a sight 1908 with crosshairs 1909 to precisely align and orient reference body and attached external fixation frame. Additionally, reference body 1900, sight 1908, and crosshairs 1909 can aid in aligning fluoroscopic images with landmarks on a patient, implant, instrument, and/or external frame. In another embodiment, the targeting center 1902 may be a sphere, pin, cylinder or ring. Marker 1902 functions as a targeting center in conjunction with crosshairs 1909 and may be made of a radiopaque material within reference body 1900. In another embodiment, marker 1902 and crosshairs 1909 may interact with a radiopaque material of the external frame 10, bone wires and/or bone pins 18, or a fastener 20 to aid in alignment and orientation.

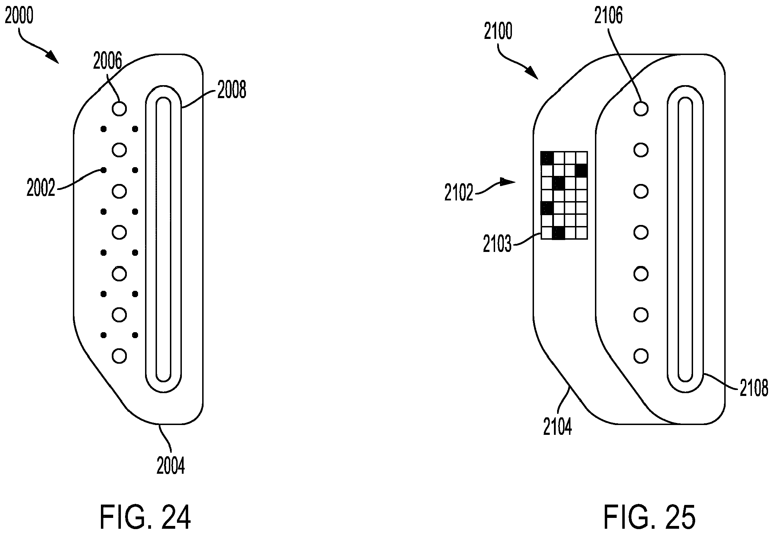

While the references bodies of the present disclosure are generally described with reference to external fixation frames, it should be understood that these reference bodies can be used in conjunction with any surgical devices to aid in visual or computer based alignment and orientation of the surgical tools. For example, FIG. 24 shows a cutting guide 2000 with multiple markers 2002 distributed throughout body 2004 for the purpose of making various types of osteotomy cuts within a digital surgical plan. A sufficient number of markers 2002 are provided and arranged in specific pattern around guide wire holes 2006 with reference to a cutting slot 2008 to allow for precise placement and alignment of cutting guide without the need for an external reference source or body.

FIG. 25 shows a cutting guide 2100 according to another embodiment of the present disclosure. Cutting guide 2100 is similar to cutting guide 2000, and therefore like elements are referred to with similar numerals within the 2100-series of numbers. For example, cutting guide includes a body 2104 with guide wire holes 2106 and cutting slot 2108. However, markers 2102 are disposed within a grid pattern 2103 as shown in FIG. 25.

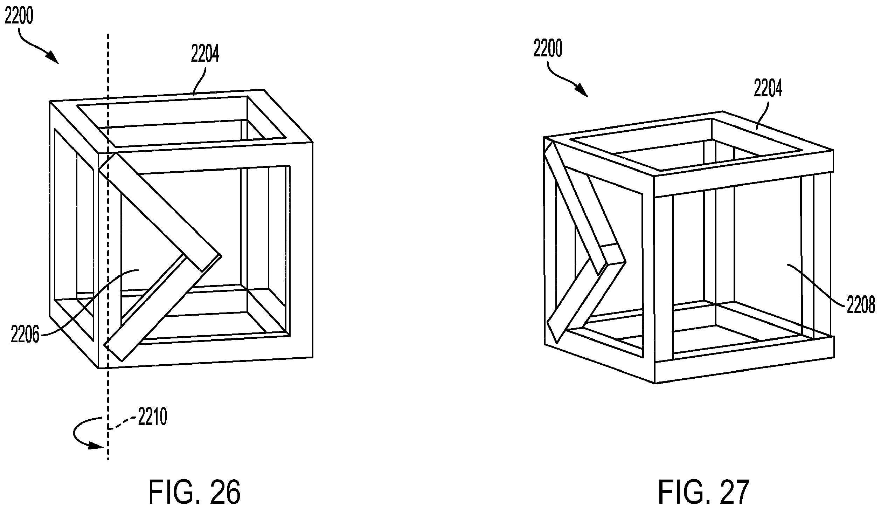

FIGS. 26 and 27 show a reference body 2200 according to another embodiment of the present disclosure. Reference body 2200 includes a framework 2204 defining a cuboidal body in this embodiment. Other embodiments may include frameworks defining other shapes such as a cylinder, pyramid, sphere, octahedron, etc. Framework 2204 can be made of radiopaque material such as metals and is arranged to define various 2D shapes such as a triangle 2206, rectangle 2208, etc. as shown in FIGS. 26 and 27. Other embodiments can include 3D shapes. In other embodiments, the framework may be made of an organic lattice constructed from a variety of different shapes and sizes. This organic lattice can be created through the use of additive manufacturing, a process in which layers of material are added in a series of steps in order to create a 3D object. This process allows for the creation of complex organic shapes in a relatively short amount of time.

The position and size of these shapes and/or the framework 2204 shown in X-ray or other imaging modules can be used to orient the external fixation frame. For example, geometric properties of these shapes such as an area defined by triangle 2206 can indicate rotation of reference body 2200 about an axis 2210 shown in FIG. 26. The area of triangle 2206 will reach its maximum when the face defining triangle 2206 of reference body 2200 is exactly perpendicular to the X-rays. Any reduction in the area of triangle 2206 will correspond to rotation of reference body 2200 around axis 2210. Additionally, geometrical properties such as area, angle, etc. of other shapes defined by framework 2204 can be observed and measured in relation to their respective axes or other reference points, in order to precisely calculate the position of reference body 2200 either manually or with a computer program. Other geometric properties such as angles, perimeter, number of sides, etc. of a shape can be used in other embodiments. This feature allows X-rays to be accurately taken at specific intervals, such as 90 degrees apart, in order to ensure that the external fixation frame is in the right orientation. Furthermore, these features can precisely detect any deviation of the X-rays from being 90 degrees apart, thus supplying the planning software with the required input to make any necessary adjustments.

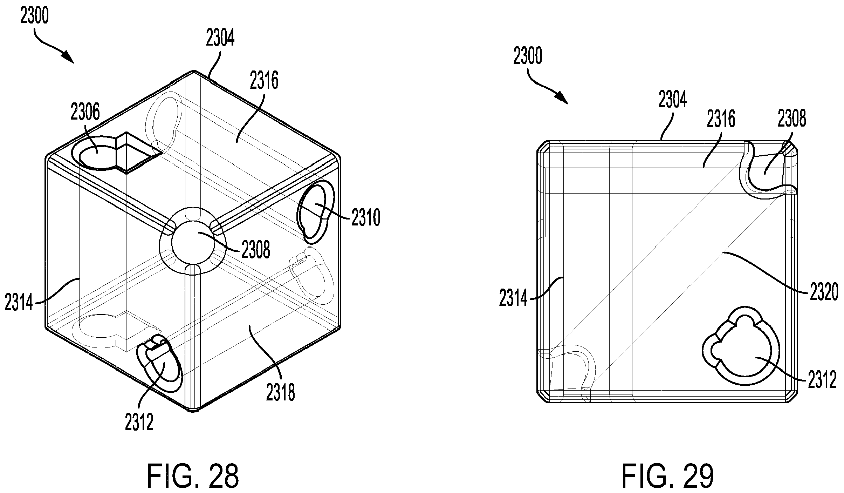

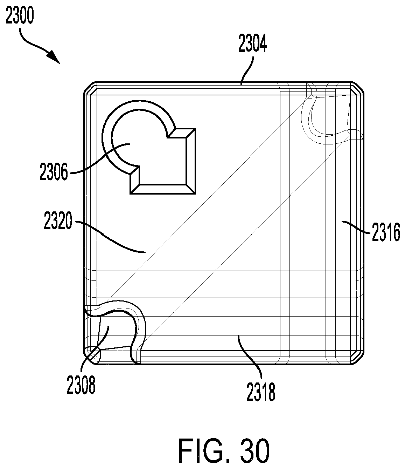

Referring now to FIGS. 28-30, there is shown a reference body 2300 according to another embodiment of the present disclosure. A reference body according to this embodiment can include a solid body 2304 with multiple through holes that function as markers to provide distinct projections when viewed on X-ray images. Solid body 2304 in this embodiment is metallic. In another embodiment, the reference body can be made of an organic lattice as described above with reference to embodiment shown in FIGS. 26 and 27. Solid body 2304 includes four through holes 2314, 2316, 2318, 2320 as shown in FIGS. 28 and 29. Three of the four through holes 2314, 2316, 2318 extend from one face to an opposite face of solid body 2304 as best shown in FIG. 28. The third through hole 2320 extends diagonally across solid body 2304 as shown in FIG. 29. Each through hole can have distinct openings. For example, opening 2306 of through hole 2314 is defined by an overlapping rectangle and a circle, opening 2310 of through hole 2310 is defined by two overlapping circles of varying diameters, opening 2312 of through hole 2318 is defined by three overlapping circles, opening 2308 of through hole 2320 is defined by a single circle.

Openings and/or through holes viewed in an X-ray provide distinctive projections depending on the incidence of the X-rays. The geometric properties of the openings and/or through holes on the X-rays such as area, angle, perimeter, etc. can be used by a software to precisely calculate the position of reference body 2300 with reference to the X-ray imager. For instance, if two X-ray images are taken in the A-P and M-L planes in order to determine the orientation of the external fixation frame and the projections of the openings and/or through holes on the X-rays appear to be slightly off, the software can use the geometric properties of the openings and/or through holes such as area, angle, perimeter, etc. to detect and quantify that variation. This information can then be used to properly adjust the position of the external fixation frame or the X-ray can be repositioned until the desired X-ray images are obtained. Additionally, the X-ray deviation value can be used by the planning software to make the required adjustments. While a reference body with four through holes is shown in this embodiment, other embodiment can have a more or fewer through holes. As an example, a reference body can have a single through hole in another embodiment.

A method for orienting external fixation frame 10 with reference to bone 11 can include the steps of positioning external fixation frame 10 about bone 11, coupling one or more of the reference bodies disclosed herein to the external fixation frame. Obtaining an image of the external fixation frame and the bone using X-ray or other imaging modules such as fluoroscopy, camera, etc. Determining an orientation of the external fixation frame with reference to the bone from a pattern in the image generated by a plurality of markers in the reference body.

Furthermore, although the invention disclosed herein has been described with reference to particular features, it is to be understood that these features are merely illustrative of the principles and applications of the present invention. It is therefore to be understood that numerous modifications, including changes in the sizes of the various features described herein, may be made to the illustrative embodiments and that other arrangements may be devised without departing from the spirit and scope of the present invention. In this regard, the present invention encompasses numerous additional features in addition to those specific features set forth in the paragraphs below. Moreover, the foregoing disclosure should be taken by way of illustration rather than by way of limitation as the present invention is defined in the examples of the numbered paragraphs, which describe features in accordance with various embodiments of the invention, set forth in the paragraphs below.

## Claims

1. An external fixation frame (10) comprising;
first and second support rings (12, 14);
one or more wires or bone pins (18) coupled to any of the first and second support rings;
one or more struts (16) extending between the first and second support rings, and
a reference body (200, 300, 400, 500, 600, 700, 900, 1000, 1100, 1200, 1300, 1400, 1500, 1600, 1700, 1800, 1900) including a plurality of markers (202, 302, 402, 502, 602, 702, 902, 1002, 1102, 1202, 1302, 1402, 1502, 1612, 1702, 1802, 1902), the reference body contacting any of the one or more wires, bone pins and struts,
wherein the plurality of markers are arranged in a pattern to identify an orientation of the external fixation frame (10).

2. The external fixation frame (10) of claim 1, wherein the orientation of the external fixation frame is with respect to an adjacent bone (11).

3. The external fixation frame (10) of any of claims 1 or 2, wherein the external fixation frame is coupled to the bone (11).

4. The external fixation frame (10) of any of claims 1-3, wherein the plurality of markers are radiopaque.

5. The external fixation frame (10) of any claims 1-4, wherein an orientation of the external fixation frame with reference to the bone in an image is determined from the pattern.

6. The external fixation frame (10) of any of claims 1-5, wherein the plurality of markers define a data code (1502).

7. The external fixation frame (10) of claim 6, wherein the data code (1502) is any of a linear barcode, a matrix code, and a Quick Response (QR) code.

8. The external fixation frame (10) of any of claims 1-6, wherein the plurality of markers are tracking markers configured to be tracked by a camera.

9. The external fixation frame (10) of any of claims 1-6, wherein a scale of the external fixation frame with reference to the bone in an image is determined from the pattern.

10. The external fixation frame (10) of any of claims 1-9, wherein the reference body (200, 400, 500, 600, 1000, 1100, 1200, 1300, 1800, 1900) defines a cuboid.

11. The external fixation frame (10) of any of claims 1-10, wherein the plurality of markers (202, 602, 702, 902, 1102, 1202, 1302, 1902) are spherical bodies disposed at least partially within the reference body.

12. The external fixation frame (10) of any of claims 1-10, wherein the plurality of markers (402, 502) are cylindrical bodies.

13. The external fixation frame (10) of claim 12, wherein the cylindrical bodies include threads (405, 505).

14. The external fixation frame (10) of claim 12, wherein a first set of cylindrical bodies extend from a first face of the cuboid and a second set of cylindrical bodies extend from a second face of the cuboid.

15. The external fixation frame (10) of any of claims 1-14, wherein the reference body (1600, 1700) includes an opening (1606, 1706) to receive the one or more wires or bone pins, the opening defining a longitudinal axis extending parallel to the one or more wires.
